Europäisches Patentamt

European Patent Office

Office européen des brevets

(19)

(11) Veröffentlichungsnummer: **0 124 167**
**B1**

(12) **EUROPÄISCHE PATENTSCHRIFT**

(45) Veröffentlichungstag der Patentschrift:
15.11.89

(21) Anmeldenummer: 84200563.9

(22) Anmeldetag: 19.04.84

(51) Int. Cl.⁴: **G 01 N 21/33**, G 01 N 33/18

(54) **Anordnung zum Prüfen einer Flüssigkeit auf Beimengungen.**

(30) Priorität: 28.04.83 DE 3315443
21.02.84 DE 3406176

(43) Veröffentlichungstag der Anmeldung:
07.11.84 Patentblatt 84/45

(45) Bekanntmachung des Hinweises auf die Patenterteilung:
15.11.89 Patentblatt 89/46

(84) Benannte Vertragsstaaten:
DE FR GB IT

(56) Entgegenhaltungen:
EP-A-0 026 093
DE-A-2 712 607
DE-A-2 715 118
GB-A-1 489 056
US-A-3 710 111
US-A-3 864 044

(73) Patentinhaber: **Philips Patentverwaltung GmbH, Wendenstrasse 35 Postfach 10 51 49, D-2000 Hamburg 1 (DE)**
(84) Benannte Vertragsstaaten: **DE**

(73) Patentinhaber: **N.V. Philips' Gloeilampenfabrieken, Groenewoudseweg 1, NL- 5621 BA Eindhoven (NL)**
(84) Benannte Vertragsstaaten: **FR GB IT**

(72) Erfinder: **Harig, Egon, Reinskamp 1, D-2000 Hamburg 74 (DE)**
Erfinder: **Martens, Gerd, Reinskamp 1, D-2000 Hamburg 74 (DE)**

(74) Vertreter: **Poddig, Dieter, Dipl.- Ing., Philips Patentverwaltung GmbH Wendenstrasse 35 Postfach 10 51 49, D-2000 Hamburg 1 (DE)**

EP 0 124 167 B1

## Beschreibung

Die Erfindung bezieht sich auf eine Anordnung zum Prüfen einer Flüssgkeit auf Beimengungen bestimmter Stoffe, insbesondere zum Prüfen von Wasser auf Beimengungen von Öl, mit einer Lichtquelle, deren zumindest Teile der Flüssigkeit durchdringendes, UV-Anteile aufweisendes Licht von einem Lichtdetektor erfaßt wird, der eine Auswerteschaltung ansteuert, die ein Alarmsignal erzeugt, wenn ein vom Lichtdetektor erzeugtes Meßsignal einen vorgegebenen Wert unterschreitet.

Eine derartige Anordnung ist bekannt aus der US-PS 37 10 111. Dort durchfließt die zu prüfende Flüssigkeit eine Glasröhre, die von einer Seite durch die Lichtquelle bestrahlt wird, und auf der entgegengesetzten Seite ist ein Lichtdetektor angeordnet. Mit dieser Anordnung werden Flüssigkeiten überwacht, die durch eine Röhre fließen.

Häufig besteht die Aufgabe, Flüssigkeiten in offenen Behältern auf Beimengungen zu prüfen, beispielsweise offene Gewässer bzw. Wasser in offenen Behältern oder Kanälen. In manchen Anwendungsfällen ist es auch möglich, daß beispielsweise Wasser noch andere Beimengungen enthält, die nicht so ungünstige Auswirkungen haben wie Öl. Solche Beimengungen können Schwebstoffe sein, die bei der bekannten Anordnung eine Dämpfung des UV-Lichtes auch ohne Vorhandensein von Ölbeimengungen bewirken.

Aufgabe der Erfindung ist es daher, eine Anordnung der eingangs genannten Art anzugeben, mittels der auch Flüssigkeiten in offenen Behältern, insbesondere offene Gewässer auf Beimengungen von Öl geprüft werden können, die schwer zugänglich sind und noch andere Beimengungen, die das Meßergebnis möglichst wenig beeinflussen sollen, enthalten können.

Diese Aufgabe wird erfindungsgemäß dadurch gelöst, daß eine Antriebsvorrichtung einen Körper, der für die UV-Anteile durchlässig ist, für die der Detektor empfindlich ist, in die zu prüfende Flüssigkeit taucht und wieder herauszieht und dabei in eine Position zwischen Lichtquelle und Lichtdetektor bringt. Dabei wird davon ausgegangen, daß die zu prüfende Flüssigkeit zumindest in dem Bereich der Prüfung eine freie, nicht von einer Leitvorrichtung wie einem Rohr begrenzte Oberfläche aufweist, sofern die zu prüfende Flüssigkeit nicht ohnehin in einem mehr oder weniger offenen Kanal fließt.

Durch das Eintauchen des Körpers in die zu prüfende Flüssigkeit wird erreicht, daß beim Herausziehen die gröberen Beimengungen wie insbesondere Schwebstoffe zurückbleiben bzw. wenig am Körper haften bleiben, während beispielsweise eine Ölbeimengung einen Film auf dem Körper erzeugt, der das Licht von der Lichtquelle zum Lichtdetektor dämpft, so daß auf diese Weise beispielsweise Ölbeimengungen erfaßt werden können.

Zweckmäßig ist es, daß der Körper ein Stab aus Quarzglas ist. Ein solcher Körper ist weitgehend unempfindlich gegen die meisten aggressiven Substanzen wie Säuren und Laugen.

Das Eintauchen und Herausziehen des Körpers kann beispielsweise durch ein motorgetriebenes Gestänge erfolgen. Eine besonders einfache Anordnung ist jedoch nach einer weiteren Ausgestaltung der Erfindung dadurch gekennzeichnet, daß der Körper an einem Faden bzw. Seil hängt, der bzw. das von einer die Antriebsvorrichtungen bildenden motorgetriebenen Seiltrommel ab- und wieder aufgewickelt wird. Dies ergibt eine sehr einfache und dennoch betriebssichere Anordnung.

Um zu gewährleisten, daß im herausgezogenen Zustand während der Meßphase der Körper sich auch richtig im Meßbereich befindet, ist es zweckmäßig, daß ein Näherungsschalter so angeordnet ist, daß er anspricht, wenn der Körper sich in der vorgesehenen Position zwischen Lichtquelle und Lichtdetektor befindet. Dieser Näherungsschalter kann dann die Antriebsvorrichtung steuern.

Eine besonders günstige Ausgestaltung ist dadurch gekennzeichnet, daß die Antriebsvorrichtung den Körper periodisch in die zu prüfende Flüssigkeit taucht. Durch das periodische Eintauchen ergibt sich sogar eine Erhöhung der Empfindlichkeit, da eine Ölbeimengung bei mehrmaligem Eintauchen eine dicker werdende Schicht auf dem Körper erzeugt, während andere im Wasser befindliche Bestandteile wie Schwebstoffe immer wieder abtropfen. Dadurch erzeugt auch eine geringe Ölbeimengung nach mehrmaligem Eintauchen eine erhebliche Dämpfung des UV-Lichtes in der Prüfanordnung.

Eine weitere vorteilhafte Ausgestaltung der Erfindung ergibt sich dadurch, daß der Lichtdetektor eine Fotoemissionsröhre ist, die eine Impulsfolge mit einer mit der Lichtintensität ansteigenden Impulsfrequenz erzeugt, die von einem am Anfang einer Meßperiode auf einen vorgegebenen ersten Wert gesetzten Impulszähler gezählt wird, der einen Alarmgeber ansteuert, wenn der Impulszähler am Ende der Meßperiode einen zweiten vorgegebenen Zählerstand nicht erreicht. Hierbei ist die Auswertung der erfaßten Meßwerte über einen Impulszähler sehr zuverlässig und genau.

In weiterer Ausgestaltung der Erfindung ist für die Steuerung der Antriebsvorrichtung ein Meßtaktgenerator vorgesehen, der den vorwärts zählenden Impulszähler zu Beginn jeder Meßperiode auf Null zurücksetzt und am Ende jeder Meßperiode eine Vergleichsanordnung ansteuert, die den Inhalt des Impulszählers mit dem vorgebbaren zweiten Wert vergleicht und an den Alarmgeber ein Auslösesignal liefert, wenn der Inhalt des Impulszählers am Ende der Meßperiode kleiner als der vorgebbare zweite Wert ist. Aufgrund der Steuerung durch einen Meßtaktgenerator arbeitet die Auswerteschaltung automatisch.

Eine weitere Ausgestaltung der Erfindung ergibt sich dadurch, daß die Vergleichsanordnung einen Vergleicher enthält, der den Zählerstand des Impulszählers mit dem vorgegebenen zweiten Wert vergleicht und der an den ersten Eingang eines UND-Gliedes ein Vergleichssignal liefert, wenn der Zählerstand kleiner als ein vorgegebener zweiter Wert ist, und daß der Meßtaktgenerator am Ende jeder Meßperiode den zweiten Eingang des UND-Gliedes ansteuert, dessen Ausgang mit dem Alarmgeber verbunden ist. Das UND-Glied ermöglicht es, auf einfache Weise zu überprüfen, ob der Vergleicher meldet, daß der Zählerstand aufgrund von durch Öl verschmutztem Wasser einen vorgegebenen Wert nicht erreicht.

Da die Fotoemissionsröhre auch bei radioaktiver Strahlung Impulse liefert, kann die erfindungsgemäße Anordnung Wasser auch auf radioaktive Beimengungen prüfen, wenn die von der Fotoemissionsröhre gelieferten Impulse bei ausgeschaltetem Licht gezählt und ausgewertet werden. Deshalb ist es zweckmäßig, daß zwischen jeweils zwei Meßperioden eine Meßpause von vorgebbarer Länge liegt, daß die Lichtquelle vom Meßtaktgenerator gesteuert nur während der Meßperioden Licht ausstrahlt und daß der am Anfang der Meßpause auf den ersten vorgegebenen Wert gesetzte Impulszähler bis zum Ende der Meßpause die vom Lichtdetektor gelieferten Impulse zählt und den Alarmgeber ansteuert, wenn der Impulszähler am Ende der Meßpause einen dritten vorgebbaren Zählerstand erreicht.

Ein Ausführungsbeispiel der Erfindung wird im folgenden anhand der Zeichnung beschrieben.

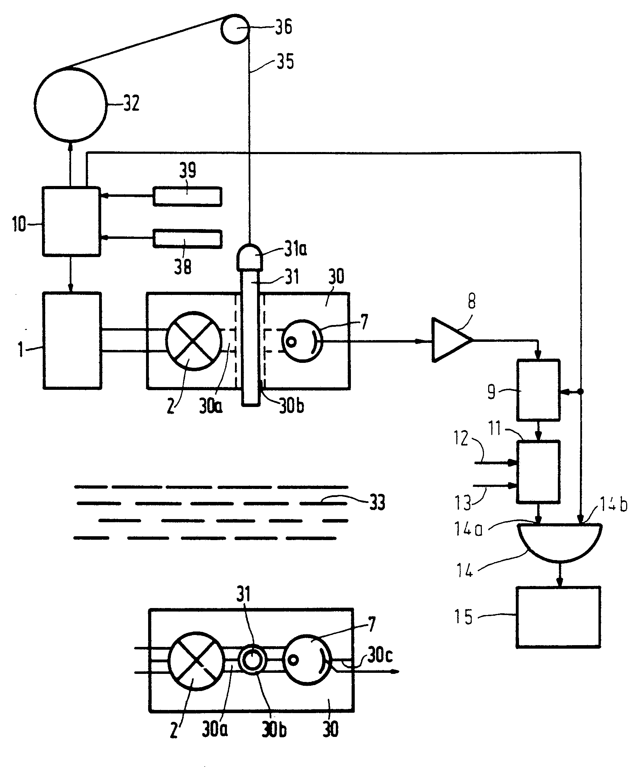

In der Figur ist eine Anordnung dargestellt, mittels der insbesondere eine sehr trübe und erhebliche Mengen von Schwebstoffen und festen Bestandteilen aufweisende Flüssigkeit wie Abwasser auf geringe Beimengungen von Öl geprüft werden kann. An einer Seiltrommel 32, die von einem nicht gesondert dargestellten Motor angetrieben wird, ist ein Seil 35 befestigt, das über eine Umlenkrolle 36 läuft. An dem anderen Ende des Seils 35 ist eine Metallkappe 31a befestigt, die einen Stab 31 aus Quarzglas hält. Der Quarzglasstab 31 befindet sich in der dargestellten Position zwischen einer Lichtquelle 2 und einem Lichtdetektor 7. Diese beiden letzteren Elemente sind in einem Gehäuse 30 angeordnet, das beispielsweise aus einem Block aus Kunststoff besteht, der einen Kanal 30b aufweist, in dem sich der Stab 31 frei bewegen kann. Ferner ist darin ein Kanal 30a zwischen der Lichtquelle 2 und dem Lichtdetektor 7 vorgesehen, der den Kanal 30b im wesentlichen senkrecht kreuzt. Zu Wartungszwecken ist der Block 30 längs der Linie 30c trennbar, wie in dem unteren Teil der Fig. 6 gezeigt ist, die einen Schnitt durch den Block 30 senkrecht zur Zeichenebene der oberen Hälfte der Figur längs des Kanals 30a darstellt. Dadurch kann beispielsweise die vordere Hälfte des Blockes 30 abgenommen werden, um die Lichtquelle 2 oder den Lichtdetektor 7 oder auch den Glasstab 31 in der herausgezogenen Position herausnehmen und ersetzen oder reinigen zu können.

Im Betrieb steuert ein Meßtaktgenerator 10 am Anfang jeder Meßperiode die Seiltrommel 32 bzw. den zugehörigen Motor an, so daß der Faden 35 abgewickelt wird und der Quarzglasstab 31 sich in die zu prüfende Flüssigkeit 33 absenkt. Wenn der Faden 35 auf geeignete Weise an der Seiltrommel 32 befestigt ist, wird nach dem vollständigen Abwickeln des Seils 35 durch das Weiterdrehen der Seiltrommel 32 das Seil 35 wieder aufgewickelt und der Quarzglasstab 31 automatisch wieder aus der Flüssigkeit 33 herausgezogen. Wenn die Metallkappe 31a bis zu einer bestimmten Nähe zum Näherungsschalter 38 herausgezogen worden ist, erzeugt letzterer ein Ausgangssignal, das dem Meßtaktgenerator 10 zugeführt wird, so daß dieser die Lichtquellensteuerung 1 ansteuert, damit die Lichtquelle 2 UV-Licht aussendet. Gleichzeitig wird die Auswertung der vom Lichtdetektor 7 erzeugten Impulse gesteuert, wie später erläutert wird. Falls sich in der Flüssigkeit 33 Anteile von Öl befunden haben, bilden diese nach dem Herausziehen des Quarzglasstabes 31 einen Film auf diesem, der das auf den Lichtdetektor 7 fallende UV-Licht der Lichtquelle 2 dämpft. Beim mehrmaligen Eintauchen des Quarzglasstabes 31 während mehrerer aufeinanderfolgender Meßperioden lagern sich mehrere Schichten von Öl auf dem Quarzglasstab 31 ab, so daß die Dämpfung steigt und somit auch geringe Anteile von Öl in dem Abwasser 33 festgestellt werden können. Dabei braucht in aufeinanderfolgenden Meßperioden lediglich die Drehrichtung der Seiltrommel 32 umgekehrt zu werden, wodurch sich in jeder Meßperiode ein Absenken des Quarzglasstabes 31 und ein darauf folgendes Herausziehen ohne Richtungsumkehr der Seiltrommel 32 innerhalb der Meßperiode ergibt. Dabei kann eine Zeitüberwachung in dem Meßtaktgenerator 10 vorgesehen werden, die überprüft, ob innerhalb einer vorgegebenen Zeitspanne nach Beginn des Absenkens der Näherungsschalter 38 ein Signal abgibt, um auf diese Weise ein Reißen des Seils 35 oder einen Ausfall des Antriebs der Seiltrommel 32 oder auch ein Klemmen des Stabes 31 festzustellen. Über dem Näherungsschalter 38 ist noch ein weiterer Näherungsschalter 39 angeordnet, der bei Versagen des Näherungsschalters 38 eine Abschaltung des Antriebsmotors der Seiltrommel 32 bewirkt, um ein Zerstören der Anordnung durch ein nicht endendes Aufwickeln des Seils 35 zu verhindern.

Das vom Lichtdetektor 7 über den Verstärker 8 an den Impulszähler 9 gelieferte Meßsignal ist eine Impulsfolge mit einer mit der Intensität des den Fotodetektor 7 treffenden Lichtes ansteigenden Impulsfrequenz.

Vom Impulszähler 9, der zu Beginn jeder Meßperiode vom Meßtaktgenerator 10 mittels eines kurzen Impulses auf Null zurückgesetzt wird, werden die von der Fotoemissionsröhre 7 gelieferten Impulse gezählt. Der Impulszähler 9 liefert ein den Zählerinhalt darstellendes analog oder digital ausgebildetes Signal an den Vergleicher 11, der dieses Signal mit einem weiteren, über den Eingang 12 eingegebenen Signal vergleicht und an den Eingang 14a des UND-Gliedes 14 ein Signal liefert, solange der Inhalt des vorwärtszählenden Impulszählers den über den Eingang 12 vorgebbaren Wert unterschreitet. Nur wenn die zu prüfende Flüssigkeit keine Beimengungen enthält, trifft genug Licht die Fotoemissionsröhre 7 und ist die Frequenz des von der Fotoemissionsröhre an den Impulszähler 9 gelieferten Meßsignales hoch genug, daß der Impulszähler 9 noch vor Ablauf der Meßperiode den eingestellten Wert erreicht und am Ende der Meßperiode kein Signal an das UND-Glied 14 liefert. Wird dann das UND-Glied 14 am Ende der Meßperiode am Eingag 14b vom Meßtaktgenerator 10 angesteuert, liegt zwar an diesem Eingang 14b ein Signal, am Eingang 14a liegt aber kein Signal, so daß vom UND-Glied 14 kein Auslösesignal an den Alarmgeber geliefert wird.

Zählt der Impulszähler 9 wegen einer Ölverschmutzung der Flüssigkeit und der daraus folgenden Lichtdämpfung zu langsam, wird am Ende der Meßperiode der vorgegebene Wert vom Impulszähler 9 nicht erreicht, und der Vergleicher 11 liefert an den Eingang 14a bis zum Ende der Meßperiode ein Vergleichssignal. Wird deshalb am Ende der Meßperiode auch vom Meßtaktgenerator 10 an den Eingang 14b ein Signal geliefert, erzeugt das UND-Glied 14 das Auslösesignal, das den Alarmgeber ansteuert und ein Alarmsignal auslöst. Das UND-Glied 14 kann auch durch ein D-Flipflop ersetzt werden, das am D-Eingang das Ausgangssignal des Vergleichers 11 und am Takteingang das Taktsignal des Meßtaktgenerators 10 erhält.

Der Impulszähler 9 kann auch von dem Impuls des Meßtaktgenerators 10 auf eine von Null verschiedene Stellung entsprechend dem Signal am Eingang 12 gesetzt werden und beispielsweise rückwärts zählen. Dann kann ein die Endstellung des Zählers angebendes Signal dem UND-Glied 14 direkt zugeführt werden, und der Vergleicher 11 ist dann derart ausgebildet, daß er dem Eingang 14a des UND-Gliedes 14 ein Signal zuführt, solange der rückwärtszählende Impulszähler 9 den über den Eingang 12 eingegebenen zweiten Wert, z.B. Null, nicht erreicht hat.

Der Meßtaktgenerator 10 liefert zwischen den zu Beginn der Meßperiode an den Impulszähler 9 und den am Ende der Meßperiode an das UND-Glied 14 gelieferten Signalen an die Lichtquellensteuerung 1 ein Signal, damit die Lichtquellensteuerung 1 die Lichtquelle 2 nur während der Meßperioden einschaltet. Zwischen den Meßperioden können längere Meßpausen liegen, in denen die Lichtquelle 2 kein Licht aussendet, um den Verschleiß der Lichtquelle 2 zu reduzieren. Der Meßtaktgenerator 10 ist zweckmäßig selbstprüfend aufgebaut, so daß bei Ausfall der Meßtakte ein entsprechendes Alarmsignal erzeugt wird, denn in diesem Falle findet keine Prüfung der Flüssigkeit mehr statt.

In den Meßpausen kann die Anordnung zum Prüfen einer Flüssigkeit auf radioaktive Beimengungen verwendet werden, da die Fotoemissionsröhre 7 auch bei radioaktiver Strahlung Impulse aussendet. Diese werden von dem zu Beginn der Meßpause vom Meßtaktgenerator 10 auf Null zurückgesetzten Impulszähler 9 gezählt. Der Vergleicher 11 ist derart ausgebildet, daß er erst dann an den Eingang 14a des UND-Gliedes 14 ein Signal liefert, wenn der Impulszähler 9 den über die Eingabemöglichkeit 13 einstellbaren dritten Wert überschreitet. Wird der dritte Wert am Ende der Meßpause überschritten, weist die Flüssigkeit radioaktive Beimengungen auf. Wenn daher am Ende der Meßperiode auch vom Meßtaktgenerator 10 an den Eingang 14b des UND-Gliedes 14 ein Impuls geliefert wird, steuert das UND-Glied 14 den Alarmgeber 15 mit einem Auslösesignal an und löst den Alarm aus.

Die beschriebene Ausführung, bei der ein Quarzglasstab an einem Seil oder einem Faden in die zu prüfende Flüssigkeit getaucht wird, hat auch den Vorteil, daß nur eine kleine Öffnung für den Zugang zu der prüfenden Flüssigkeit erforderlich ist und daß die eigentliche Prüfanordnung mit Lichtquelle, Lichtdetektor und Auswertungselektronik weit von der zu prüfenden Flüssigkeit entfernt angebracht werden kann. Insbesondere kann beispielsweise tiefliegendes Grundwasser durch ein enges Bohrloch leicht auf Ölbeimengungen überwacht werden. Da die Prüfanordnung sich im Bereich der Erdoberfläche befindet, wird eine Wartung wesentlich vereinfacht. Wenn diese räumliche Trennung von zu prüfender Flüssigkeit und Prüfanordnung nicht erforderlich ist, kann zur Prüfung einer mit erheblichen Mengen von Schwebstoffen oder größeren festen Teilen versetzten Flüssigkeit auch eine im wesentlichen runde Quarzscheibe verwendet werden, die um eine parallel zur Flüssigkeitsoberfläche liegende Achse gedreht wird und zum Teil in die Flüssigkeit taucht. Ein außerhalb der Flüssigkeit liegendes Teil der Quarzscheibe läuft dann zwischen Lichtsender und Lichtempfänger hindurch. Auch auf diese Weise wird erreicht, daß ein Körper, nämlich jeder äußere Teil der Quarzscheibe, periodisch in die zu prüfende Flüssigkeit getaucht und wieder herausgezogen wird.

## Patentansprüche

1. Anordnung zum Prüfen einer Flüssigkeit auf Beimengungen bestimmter Stoffe, insbesondere zum Prüfen von Wasser auf Beimengungen von Öl, mit einer Lichtquelle, deren zumindest Teile der Flüssigkeit durchdringendes, UV-Anteile aufweisendes Licht von einem Lichtdetektor erfaßt wird, der eine Auswerteschaltung ansteuert, die ein Alarmsignal erzeugt, wenn ein vom Lichtdetektor erzeugtes Meßsignal einen vorgegebenen Wert unterschreitet,
dadurch gekennzeichnet, daß eine Antriebsvorrichtung (32) einen Körper (31), der für die UV-Anteile durchlässig ist, für die der Detektor (7) empfindlich ist, in die zu prüfende Flüssigkeit (33) taucht und wieder herauszieht und dabei in eine Position zwischen Lichtquelle (2) und Lichtdetektor (7) bringt.

2. Anordnung nach Anspruch 1,
dadurch gekennzeichnet, daß der Körper (31) ein Stab aus Quarzglas ist.

3. Anordnung nach Anspruch 1 oder 2,
dadurch gekennzeichnet, daß der Körper (31) an einem Faden bzw. Seil (35) hängt, der bzw. das von einer die Antriebsvorrichtung (32) bildenden motorgetriebenen Seiltrommel ab- und wieder aufgewickelt wird.

4. Anordnung nach einem der Ansprüche 1, 1 oder 3,
dadurch gekennzeichnet, daß ein Näherungsschalter (38) so angeordnet ist, daß er anspricht, wenn der Körper (31) sich in der vorgesehenen Position zwischen Lichtquelle (2) und Lichtdetektor (7) befindet.

5. Anordnung nach einem der Ansprüche 1 bis 4,
dadurch gekennzeichnet, daß die Antriebsvorrichtung (32) den Körper (31) periodisch in die zu prüfende Flüssigkeit (33) taucht.

6. Anordnung nach Anspruch 5,
dadurch gekennzeichnet, daß der Lichtdetektor (7) eine Fotoemissionsröhre ist, die eine Impulsfolge mit einer mit der Lichtintensität ansteigenden Impulsfrequenz erzeugt und einem am Anfang einer Meßperiode auf einen vorgebbaren ersten Wert gesetzten Impulszähler (9) zuführt, der einen Alarmgeber (15) ansteuert, wenn der Impulszähler (9) am Ende der Meßperiode einen zweiten vorgegebenen Zählerstand nicht erreicht.

7. Anordnung nach Anspruch 6,
dadurch gekennzeichnet, daß für die Steuerung der Antriebsvorrichtung (32) ein Meßtaktgenerator (10) vorgesehen ist, der den vorwärtszählenden Impulszähler (9) zu Beginn jeder Meßperiode auf Null zurücksetzt und am Ende jeder Meßperiode eine Vergleichsanordnung (11 bis 14) ansteuert, die den Inhalt des Impulszählers (9) mit dem vorgebbaren zweiten Wert vergleicht und an den Alarmgeber (15) ein Auslösesignal liefert, wenn der Inhalt des Impulszählers (9) am Ende der Meßperiode kleiner als der vorgebbare zweite Wert ist.

8. Anordnung nach Anspruch 7,
dadurch gekennzeichnet, daß die Vergleichsanordnung (11, 14) einen Vergleicher (11) enthält, der den Zählerstand des Impulszählers (9) mit dem vorgegebenen zweiten Wert vergleicht und der an den ersten Eingang (14a) eines UND-Gliedes (14) ein Vergleichssignal liefert, wenn der Zählerstand kleiner als ein vorgegebener zweiter Wert ist, und daß der Meßtaktgenerator (10) am Ende jeder Meßperiode den zweiten Eingang (14b) des UND-Gliedes (14) ansteuert, dessen Ausgang mit dem Alarmgeber (15) verbunden ist.

9. Anordnung nach einem der Ansprüche 5 bis 8,
dadurch gekennzeichnet, daß zwischen jeweils zwei Meßperioden eine Meßpause von vorgebbarer Länge liegt, daß die Lichtquelle (2) vom Meßtaktgenerator (20) gesteuert nur während der Meßperiode Licht ausstrahlt, und daß der am Anfang der Meßpause auf den ersten vorgegebenen Wert gesetzte Impulszähler (9) bis zum Ende der Meßpause die vom Lichtdetektor (7) gelieferten Impulse zählt und den Alarmgeber (15) ansteuert, wenn der Impulszähler (9) am Ende der Meßpause einen dritten vorgebbaren Zählerstand erreicht.

## Claims

1. An apparatus for determining in a liquid the presence of admixtures of given materials, more particularly for determining in water the presence of admixtures of oil, comprising a light source, whose light penetrating at least into parts of the liquid and containing ultraviolet components is detected by a photo detector, which drives an evaluation circuit, which produces an alarm signal when a measuring signal produced by the photo detector falls below a given value, characterized in that a driving device (32) immerses a body (31), which is transparent to the ultraviolet components, to which the detector (7) is sensitive, into the liquid (33) to be tested and withdraws it again and brings it into a position between the light source (2) and the light detector (7).

2. An apparatus as claimed in Claim 1, characterized in that the body (31) is a rod of quartz glass.

3. An apparatus as claimed in Claim 1 or 2, characterized in that the body (31) is suspended from a thread or rope (35), which is wound off and rewound onto a rope drum constituting the driving device (32) and driven by a motor.

4. An apparatus as claimed in Claim 1, 2 or 3, characterized in that a proximity switch (38) is arranged so that it responds when the body (31) is in the given position between the light source (2) and the photo detector (7).

5. An apparatus as claimed in any one of Claims 1 to 4, characterized in that the driving device (32) periodically immerses the body (31) into the liquid (33) to be tested.

6. An apparatus as claimed in Claim 5, characterized in that the photo detector (7) is a photoemission tube, which produces a pulse sequence having a pulse frequency increasing with the light intensity and supplies it to a pulse counter (9), which is set at the beginning of a measuring period to a given first value and drives an alarm device (15) when the pulse counter (9) does not reach at the end of the measuring period a second given count.

7. An apparatus as claimed in Claim 6, characterized in that the driving device (32) is controlled by a measuring clock generator (10), which resets the positively counting pulse counter (9) to zero at the beginning of each measuring period and drives at the end of each measuring period a comparison device (11 to 14), which compares the content of the pulse counter (9) with the given second value and supplies a triggering signal to the alarm device (15) when the content of the pulse counter (9) at the end of the measuring period is smaller than the given second value.

8. An apparatus as claimed in Claim 7, characterized in that the comparison device (11, 14) comprises a comparator (11), which compares the count of the pulse counter (9) with the given second value and supplies a comparison signal to the first input (14a) of an AND member (14) when the count is smaller than a given second value, and in that the measuring clock generator (10) drives at the end of each measuring period the second input (14b) of the AND member (14), whose output is connected to the alarm device (15).

9. An apparatus as claimed in any one of Claims 5 to 8, characterized in that between each time two measuring periods a measuring interval is provided of a given length, in that the light source (2) under the control of the measuring clock generator (10) emits light only during the measuring period, and in that the pulse counter (9) set to the first given value at the beginning of the measuring interval counts the pulses supplied by the photodetector (7) till the end of the measuring interval and drives the alarm device (15) when the pulse counter (9) reaches at the end of the measuring interval a third given count.

## Revendications

1. Système pour contrôler un liquide en vue d'y déceler des adjonctions de certaines substances, en particulier pour vérifier la présence d'adjonctions de pétrole dans l'eau, comportant une source lumineuse dont la lumière, comprenant une fraction UV traversant au moins des parties du liquide, est détectée par un détecteur de lumière qui excite un circuit d'évaluation produisant un signal d'alarme lorsqu'un signal de mesure engendré par le détecteur de lumière est en deçà d'une valeur prédéfinie,

caractérisé en ce qu'un dispositif d'entraînement (32) plonge, dans le liquide à contrôler (33), un corps (31) qui est transparent à l'égard de la fraction UV à laquelle le détecteur (7) est sensible, et l'en retire ensuite pour l'amener dans une position située entre la source lumineuse (2) et le détecteur de lumière (7).

2. Système suivant la revendication 1, caractérisé en ce que le corps (31) est un barreau en verre-quartzeux.

3. Système suivant la revendication 1 ou 2, caractérisé en ce que le corps (31) est suspendu à un fil ou un câble (35) qui est dévidé et renvidé par un tambour à câble à moteur constituant le dispositif d'entraînement (32).

4. Système suivant l'une des revendications 1 à 3,

caractérisé en ce qu'un détecteur de proximité (38) est disposé de manière à réagir lorsque le corps (31) se trouve dans la position prévue entre la source lumineuse (2) et le détecteur de lumière (7).

5. Système suivant l'une quelconque des revendications 1 à 4,

caractérisé en ce que le dispositif d'entraînement (32) plonge le corps (31) périodiquement dans le liquide (33) à contrôler.

6. Système suivant la revendication 5, caractérisé en ce que le détecteur de lumière (7) est un tube photoémetteur qui produit une séquence d'impulsions à une fréquence d'impulsions augmentant avec l'intensité lumineuse et l'applique à un compteur d'impulsions (9) mis à une première valeur prédéfinie au début d'une période de mesure, qui excite un générateur d'alarme (15) lorsqu'il n'atteint pas, à la fin de la période de mesure, un second état de comptage prédéfini.

7. Système suivant la revendication 6, caractérisé en ce que pour la commande du dispositif d'entraînement (32) est prévu un générateur de rythme de mesure (10) qui, au début de chaque période de mesure, remet le compteur d'impulsions progressif (9) à zéro et, à la fin de chaque période de mesure, excite un dispositif comparateur (11 à 14) qui compare le contenu du compteur d'impulsions (9) avec la deuxième valeur prédéfinie et fournit un signal d'enclenchement au générateur d'alarme (15) lorsque le contenu du compteur d'impulsions (9), à la fin de la période de mesure, est inférieur à la seconde valeur prédéfinie.

8. Système suivant la revendication 7, caractérisé en ce que le dispositif comparateur (11, 14) contient un comparateur (11) qui compare l'état de comptage du compteur d'impulsions (9) avec la seconde valeur prédéfinie et qui fournit un signal de comparaison à la première entrée (14a) d'un élément ET (14), lorsque l'état de comptage est inférieur à une seconde valeur prédéfinie, et que le générateur de rythme de mesure (10) excite, à la fin de chaque période de mesure, la seconde entrée (14b) de l'élément ET, dont la sortie est connectée au générateur d'alarme (15).

9. Système suivant l'une quelconque des revendications 5 à 8,

caractérisé en ce qu'une pause de mesure de longueur prédéfinie est prévue entre chaque fois deux périodes de mesure, que la source lumineuse (2), sous la commande du générateur de rythme de mesure (10), n'émet de la lumière que pendant la période de mesure et que le compteur d'impulsions (9) mis à la première valeur prédéfinie au début de la pause de mesure compte les impulsions fournies par le détecteur de lumière (7) jusqu'à la fin de la pause de mesure et excite le générateur d'alarme (15) lorsque le compteur d'impulsions (9) atteint, à la fin de la pause de mesure, un troisième état de comptage prédéfini.